# EUROPÄISCHE PATENTANMELDUNG

(11) **EP 1 243 642 A2**
(43) Veröffentlichungstag der Anmeldung: **25.09.2002**
(21) Anmeldenummer: 02005262.7
(22) Anmeldetag: 11.03.2002
(51) Int. Cl.: C11B 9/00, C07C 67/08, C07C 69/007, C07C 69/24

(54) **Verfahren zur Herstellung von Alkylcarbonsäureallylestern**

(30) Priorität: 22.03.2001 DE 10113964
(71) Anmelder: HAARMANN & REIMER GMBH, 37603 Holzminden (DE)
(72) Erfinder: Kuhn, Walter, Dr., 37603 Holzminden (DE); Senft, Gerhard, 37603 Holzminden (DE)
(74) Vertreter: Mann, Volker, Dr.

(57) **Zusammenfassung**

Die vorliegende Erfindung betrifft ein Verfahren zur Herstellung von Alkylcarbonsäureallylestern durch Umsetzung von Allylalkohol und Alkylcarbonsäuren.

## Beschreibung

Die vorliegende Erfindung betrifft ein Verfahren zur Herstellung von Alkylcarbonsäureallylestern durch Umsetzung von Allylalkohol und Alkylcarbonsäuren.

Alkylcarbonsäureallylester sind begehrte Riechstoffe. In S. Arctander, Perfume and Flavour Chemicals, No. 70, 71, 85, 77, 88; 1969 sind die Ester Allylhexanoat, Allylheptanoat, Allyloctanoat, Allylcyclohexylpropionat und Allylnonanoat beschrieben. Diese Ester zeichnen sich durch ihren fruchtigen, ananasartigen Charakter aus. Der direkte Weg zu Alkylcarbonsäureallylestern ist durch die azeotrope, säurekatalysierte Veresterung von Allylalkohol (2-Propen-1-ol) und der entsprechenden Alkylcarbonsäure unter Zuhilfenahme eines Wasserschleppers gegeben. Als Wasserschlepper werden üblicherweise mit Wasser azeotropbildende Lösungsmittel wie z.B. Benzol, Toluol, Hexan oder Cyclohexan eingesetzt.

In Journal of Perfumer and Flavorist, Vol. 25, Januar-Februar 2000, S. 20 wird die Umsetzung von Allylalkohol und Cyclohexylpropionsäure zu Allylcyclohexylpropionat mittels n-Hexan als Wasserschlepper und para-Toluolsulfonsäure als Katalysator beschrieben. Allylalkohol wird dabei in 5-fachem Überschuss eingesetzt.

Der Einsatz der genannten azeotropbildenden Lösungsmittel ist unerwünscht. Diese können ebenfalls den sensorischen Eindruck negativ beeinflussen. Ein erhöhter Reinigungsaufwand ist daher zur Abtrennung des Wasserschleppers erforderlich.

Es bestand daher die Aufgabe, ein Verfahren zur Herstellung von Alkylcarbonsäureallylestern zu finden, das einen einfachen Weg zu sensorisch einwandfreien Alkylcarbonsäureallylestern ermöglicht.

Die vorliegende Erfindung betrifft ein Verfahren zur Herstellung von Alkylcarbonsäureallylestern durch Umsetzung einer Alkylcarbonsäure mit Allylalkohol in Gegenwart einer Katalysatorsäure, dadurch gekennzeichnet, dass zu Beginn der Reaktion ein Alkylcarbonsäureallylester zugesetzt wird.

Eine bevorzugte Ausführungsform ist dadurch gekennzeichnet, dass kein zusätzliches als Wasserschlepper fungierendes Lösungsmittel zugesetzt wird. Eine besonders bevorzugte Ausführungsform ist dadurch gekennzeichnet, dass vor Reaktionsbeginn dem Gemisch aus Alkylcarbonsäure und Katalysatorsäure der zu bildende Alkylcarbonsäureallylester zugesetzt wird.

Ein weiterer Gegenstand der vorliegenden Erfindung ist ein Verfahren zur Herstellung von Alkylcarbonsäureallylestern durch Umsetzung einer Alkylcarbonsäure mit Allylalkohol in Gegenwart einer Katalysatorsäure dadurch gekennzeichnet, dass kein zusätzliches als Wasserschlepper fungierendes Lösungsmittel eingesetzt wird.

Diallylether kann ebenfalls vor Beginn der Reaktion zugesetzt werden. Diallylether kann bei der Veresterungsreaktion als Nebenprodukt entstehen und im abdestillierenden Azeotrop enthalten sein.

Die nach den genannten Verfahren hergestellten Alkylcarbonsäureallylester finden vor allem Verwendung als Riechstoffe, in Parfümkompositionen, Parfümölen oder Duftkompositionen.

Bevorzugte erfindungsgemäß geeignete Alkylcarbonsäuren entsprechen der Formel R¹-COOH, wobei R¹ für geradkettige, verzweigte oder cyclische Alkylreste stehen kann. Bevorzugt sind Alkylreste R¹ mit 3 bis 12 Kohlenstoffatomen. Als Beispiel für eine Cycloalkylcarbonsäure sei 3-Cyclohexylpropionsäure genannt.

Für R¹ seien beispielsweise genannt: n-Propyl, iso-Propyl, n-Butyl, 2-Butyl, isoButyl, n-Pentyl, iso-Pentyl, 2-Pentyl, 3-Pentyl, n-Hexyl, neo-Hexyl, 2-Methylpentyl, 3-Methylpentyl, 4-Methylpentyl, 2,3-Dimethylbutyl, n-Heptyl, 2-Methylhexyl, 3-Methylhexyl, 4-Methylhexyl, 5-Methylhexyl, 2,3-Dimethylpentyl, 2,4-Dimethylpentyl, 3,4-Dimethylpentyl, 3,3-Dimethylpentyl, 4,4-Dimethylpentyl, n-Heptyl, iso-Heptyl, n-Octyl, iso-Octyl, n-Nonyl, 2-Ethylhexyl, 2,3-Diethylpentyl, 2,4-Diethylpentyl, 3,4-Diethylpentyl, 2-Ethylheptyl, 3-Ethylheptyl, 4-Ethylheptyl, 2-Ethyloctyl, 3-Ethyloctyl, 4-Ethyloctyl, 5-Ethyloctyl, 3-Ethyl-2-methylhexyl, 2-Ethyl-3-methylhexyl, 2-Ethyl-5-methylhexyl, 3-Ethyl-5-methylhexyl, Cyclopropyl, Cyclobutyl, Cyclopentyl, 2-Methylcyclopentyl, 3-Methylcyclopentyl, 4-Methylcyclopentyl, 2,3-Dimethylcyclopentyl, 2,4-Dimethylcyclopentyl, 3,3-Dimethylcyclopentyl, 4,4-Dimethylcyclopentyl, 2,3-Diethylcyclopentyl, 2,4-Diethylcyclopentyl, 2-Ethyl-3-methylcyclopentyl, 3-Ethyl-2-methylcyclopentyl, 3-Ethyl-4-methylcyclopentyl, 4-Ethyl-3-methylcyclopentyl, Cyclohexyl, 2-Methylcyclohexyl, 3-Methylcyclohexyl, 4-Methylcyclohexyl, 2-Ethylcyclohexyl, 3-Ethylcyclohexyl, 4-Ethylcyclohexyl, 5-Ethylcyclohexyl, 2-Cyclohexylethyl, 3-Cyclohexylpropyl, 3-Cyclohexylbutyl, 4-Cyclohexylbutyl, Cyloheptyl, Cylononyl, Cyclodecyl, Cyclododecyl.

Erfindungsgemäß werden als Alkylcarbonsäure bevorzugt Pentansäure, Hexansäure, Heptansäure, Octansäure, Cyclohexylpropionsäure oder Nonansäure verwendet.

Besonders bevorzugt sind n-Pentansäure, n-Hexansäure, n-Heptansäure, n-Octansäure, 3-Cyclohexylpropionsäure oder n-Nonansäure.

Als Katalysatorsäure eignen sich starke Säuren wie Schwefelsäure, Phosphorsäure, p-Toluolsulfonsäure oder Salzsäure.

Die Umsetzung wird erfindungsgemäß bei 70-170°C, bevorzugt bei 120-140°C durchgeführt.

Das erfindungsgemäße Verfahren kann in einem weiten Druckbereich durchgeführt werden, bevorzugt ist eine Reaktionsführung bei Normaldruck.

Die folgenden Mengenangaben und Verhältnisse beziehen sich auf molare Mengen und Angaben.

Die geeignete Einsatzmenge Katalysatorsäure bezogen auf die Einsatzmenge an Alkylcarbonsäure liegt im Bereich 0,0001 bis 0,05 : 1, bevorzugt im Bereich 0,001 bis 0,01: 1.

Das Verhältnis von Allylalkohol bezogen auf die Alkylcarbonsäure kann in weiten Bereichen von 0,1 : 1 bis 10 : 1 liegen, geeigneterweise im Bereich von 0,5 : 1 bis 5 : 1. Bevorzugt wird Allylalkohol im Überschuss im Bereich 1 bis 2 : 1, besonders bevorzugt im Bereich 1,1 bis 1,5 : 1 eingesetzt.

Wird die Umsetzung der Alkylcarbonsäure mit Allylalkohol in Abwesenheit eines Alkylcarbonsäureallylesters durchgeführt, so verläuft die Umsetzung unter gleichen Reaktionsbedingungen etwas langsamer und der Gehalt an Alkylcarbonsäureallylester nach der Reaktion ist zumeist niedriger als bei Zusatz eines Alkylcarbonsäureallylesters.

Wird ein Alkylcarbonsäureallylester mit in die Reaktion vorgelegt, so kann dies in weiten Bereichen erfolgen. Bezogen auf die molare Menge an eingesetzter Alkylcarbonsäure kann der zugesetzte Alkylcarbonsäureallylester im Verhältnis 0.001 bis 0,8 : 1, bevorzugt im Verhältnis 0,01 bis 0,03 : 1 zugesetzt werden.

In einer bevorzugten Ausführungsform wird der zu bildende Alkylcarbonsäureallylester zugesetzt, besonders bevorzugt vor Reaktionsbeginn.

Erfindungsgemäß werden als Alkylcarbonsäureallylester bevorzugt Allylpentanoat, Allylhexanoat, Allylheptanoat, Allyloctanoat, Allylcyclohexylpropionat oder Allylnonanoat eingesetzt.

Besonders bevorzugt sind n-Pentansäureallylester, n-Hexansäureallylester, n-Heptansäureallylester, n-Octansäureallylester, Cyclohexylpropionsäureallylester oder n-Nonansäureallylester.

Das erfindungsgemäße Verfahren kann beispielsweise folgendermaßen durchgeführt werden:
Allylalkohol wird in die vorgelegte Alkylcarbonsäure R¹-COOH und Katalysatorsäure bzw. einem Gemisch aus Alkylcarbonsäure R¹-COOH, Katalysatorsäure und gegebenenfalls einem Alkylcarbonsäureallylester, bevorzugt dem zu bildenden Alkylcarbonsäureallylester R¹-COO-CH₂-CH=CH₂, eindosiert. Gleichzeitig wird das Destillat, das im wesentlichen aus Wasser, Allylalkohol und Alkylcarbonsäureallylester besteht, über einen Wasserabscheider geführt. Wasser wird dabei kontinuierlich abgetrennt. Gegebenenfalls kann eine weitere Menge an Allylalkohol zum Reaktionsansatz zugegeben werden, um den Umsatz der Reaktion zu vervollständigen. Nach Reaktionsende können Waschvorgänge folgen und das Produkt kann durch einfache Destillation erhalten werden.

### Folgende Beispiele erläutern die Erfindung:

### Beispiel 1

### Herstellung von n-Hexansäureallylester

2340 g (20,2 mol) n-Hexansäure, 50 g (0,3 mol) n-Hexansäureallylester und 19 g (0,1 mol) p-Toluolsulfonsäure werden bei 140°C vorgelegt. Es werden 1150 g (19,8 mol) Allylalkohol bei 130-140°C über 7 Stunden eindosiert. Gleichzeitig wird über einen Wasserabscheider das Azeotrop abdestilliert. Es werden 480 g an wässriger Phase abgetrennt. Nach Ende der Wasserabscheidung werden weitere 150 g (2,6 mol) Allylalkohol bei 130-140°C über 1 Stunde eindosiert und gleichzeitig 319 g an Azeotrop abdestilliert. Gegen Ende der Dosierung wird im Vakuum bei 300 mbar und bei 120°C andestilliert. Nach Abkühlen des Reaktionsansatzes wird mit 200 g Wasser und anschließend mit 600 g 5%iger NaOH-Lösung gewaschen.

3298 g Rohprodukt ergeben nach einfacher Destillation bei 78°C/32 mbar 3075 g n-Hexansäureallylester mit einer Reinheit von >99 %.

Die Ausbeute der Theorie an n-Hexansäureallylester bezogen auf Einsatz n-Hexansäure liegt bei 96 %.

### Beispiel 2

### Herstellung von n-Heptansäureallylester

2380 g (18,3 mol) n-Heptansäure, 50 g (0,3 mol) n-Heptansäureallylester und 19 g (0,1 mol) p-Toluolsulfonsäure werden bei 140°C vorgelegt. Es werden 1150 g (19,8 mol) Allylalkohol bei 130-140°C über 7 Stunden eindosiert. Gleichzeitig wird über einen Wasserabscheider das Azeotrop abdestilliert. Es werden 438 g an wässriger Phase abgetrennt. Nach Ende der Wasserabscheidung werden weitere 150 g Allylalkohol (2,6 mol) bei 130-140°C über 1 Stunde eindosiert. Gegen Ende der Dosierung wird im Vakuum bei 300 mbar und bei 120°C andestilliert. Es destillieren 273 g an Azeotrop über.

Nach Abkühlen des Reaktionsansatzes wird mit 200 g Wasser und anschließend mit 600 g 5%iger NaOH-Lösung gewaschen. 3182 g Rohprodukt ergeben nach einfacher Destillation bei 91°C/21 mbar 2943 g n-Heptansäureallylester mit einer Reinheit von >99 %.

Die Ausbeute der Theorie an n-Heptansäureallylester bezogen auf Einsatz n-Heptansäure liegt bei 93 %.

### Beispiel 3

### Herstellung von n-Hexansäureallylester ohne Zugabe von Alkylcarbonsäureallylester

Es wurde eine Umsetzung wie in Beispiel 1 beschrieben durchgeführt, wobei auf eine Zugabe von n-Hexansäureallylester verzichtet wurde. Das erhaltene Rohprodukt enthielt vor Destillation 86 % des gewünschten n-Hexansäureallylesters.

## Patentansprüche

1. Verfahren zur Herstellung von Alkylcarbonsäureallylestern durch Umsetzung einer Alkylcarbonsäure mit Allylalkohol in Gegenwart einer Katalysatorsäure, **dadurch gekennzeichnet, dass** zu Beginn der Reaktion ein Alkylcarbonsäureallylester zugesetzt wird.

2. Verfahren nach Anspruch 1, **dadurch gekennzeichnet, dass** kein zusätzliches als Wasserschlepper fungierendes Lösungsmittel zugesetzt wird.

3. Verfahren nach mindestens einem der Ansprüche 1 oder 2, **dadurch gekennzeichnet, dass** eine Alkylcarbonsäure R¹-COOH eingesetzt wird, wobei der Alkylrest R¹ 3 bis 12 Kohlenstoffatomen enthält und dieser geradkettig, verzweigt oder cyclisch sein kann.

4. Verfahren nach mindestens einem der Ansprüche 1 bis 3, **dadurch gekennzeichnet, dass** als Alkylcarbonsäure n-Pentansäure, n-Hexansäure, n-Heptansäure, n-Octansäure, 3-Cyclohexylpropionsäure oder n-Nonansäure verwendet wird.

5. Verfahren nach mindestens einem der Ansprüche 1 bis 4, **dadurch gekennzeichnet, dass** die molare Einsatzmenge von Allylalkohol bezogen auf Alkylcarbonsäure im Bereich 0,1 : 1 bis 10 : 1 liegt.

6. Verfahren nach mindestens einem der Ansprüche 1 bis 5, **dadurch gekennzeichnet, dass** die molare Einsatzmenge von Allylalkohol bezogen auf Alkylcarbonsäure im Bereich 1 : 1 bis 2 : 1 liegt.

7. Verfahren nach mindestens einem der Ansprüche 1 bis 6, **dadurch gekennzeichnet, dass** vor Reaktionsbeginn dem Gemisch aus Alkylcarbonsäure und Katalysatorsäure der zu bildende Alkylcarbonsäureallylester zugesetzt wird.

8. Verfahren nach mindestens einem der Ansprüche 1 bis 7, **dadurch gekennzeichnet, dass** als Alkylcarbonsäureallylester n-Pentansäureallylester, n-Hexansäureallylester, n-Heptansäureallylester, n-Octansäureallylester, 3-Cyclohexylpropionsäureallylester oder n-Nonansäureallylester verwendet wird.

9. Verfahren nach mindestens einem der Ansprüche 1 bis 8, **dadurch gekennzeichnet, dass** die molare Einsatzmenge des zugesetzten Alkylcarbonsäureallylesters bezogen auf Alkylcarbonsäure im Bereich 0,001 bis 0,8 : 1 liegt.

10. Verfahren nach mindestens einem der Ansprüche 1 bis 9, **dadurch gekennzeichnet, dass** die molare Einsatzmenge des zugesetzten Alkylcarbonsäureallylesters bezogen auf Alkylcarbonsäure im Bereich 0,01 bis 0,3 : 1 liegt.

11. Verfahren nach mindestens einem der Ansprüche 1 bis 10, **dadurch gekennzeichnet, dass** die Umsetzung bei einer Temperatur im Bereich 70-170°C durchgeführt wird.

12. Verfahren nach mindestens einem der Ansprüche 1 bis 11, **dadurch gekennzeichnet, dass** die Umsetzung bei einer Temperatur im Bereich 120-140°C durchgeführt wird.

13. Verfahren nach mindestens einem der Ansprüche 1 bis 12, **dadurch gekennzeichnet, dass** vor Reaktionsbeginn dem Gemisch aus Alkylcarbonsäure und Katalysatorsäure Diallylether zugesetzt wird.

14. Verfahren zur Herstellung von Alkylcarbonsäureallylestern durch Umsetzung einer Alkylcarbonsäure mit Allylalkohol in Gegenwart einer Katalysatorsäure **dadurch gekennzeichnet, dass** kein zusätzliches als Wasserschlepper fungierendes Lösungsmittel eingesetzt wird.

15. Verfahren nach Anspruch 14, **dadurch gekennzeichnet, dass** als Alkylcarbonsäure n-Pentansäure, n-Hexansäure, n-Heptansäure, n-Octansäure, 3-Cyclohexylpropionsäure oder n-Nonansäure verwendet wird.

16. Verfahren nach mindestens einem der Ansprüche 14 oder 15, **dadurch gekennzeichnet, dass** die molare Einsatzmenge von Allylalkohol bezogen auf Alkylcarbonsäure im Bereich 0,1 : 1 bis 10 : 1 liegt.

17. Verfahren nach mindestens einem der Ansprüche 14 bis 16, **dadurch gekennzeichnet, dass** die Umsetzung bei einer Temperatur im Bereich 70-170°C durchgeführt wird.

18. Verwendung der nach mindestens einem der Ansprüche 1 bis 17 hergestellten Alkylcarbonsäureallylester als Riechstoffe.

19. Parfümkompositionen, Parfümöle oder Duftkompositionen enthaltend Alkylcarbonsäureallylester, welche nach mindestens einem der Ansprüche 1 bis 17 hergestellt wurden.
